# FASCICULE DE BREVET EUROPEEN

(11) **EP 3 253 861 B1**
(45) Date de publication et mention de la délivrance du brevet: **07.08.2019**
(21) Numéro de dépôt: 16705048.3
(22) Date de dépôt: 02.02.2016
(51) Int. Cl.: C12M 1/12, A61L 27/56, C12N 5/00

(54) **MATRICE SOUS FORME DE BILLE COMME SUPPORT CELLULAIRE**
MATRIX IN KUGELFORM ALS ZELLTRÄGER
MATRIX IN BALL FORM AS A CELL CARRIER

(30) Priorité: 03.02.2015 FR 1500195
(43) Date de publication de la demande: 13.12.2017
(73) Titulaire: Maco Pharma, 59420 Mouvaux (FR)
(72) Inventeur: BOUCKENOOGHE, Thomas, 59370 Mons-en-Baroeul (FR); BERTHOLET, Pauline, 59250 Halluin (FR); DELORME, Bruno, 59700 Marcq en Baroeul (FR)
(74) Mandataire: Herrou, Nathalie
(86) Numéro de dépôt international: PCT/EP2016/052137
(87) Numéro de publication internationale: WO 2016/124569

(56) Documents cités:
- WO-A1-99/15637
- MARIANA B. OLIVEIRA ET AL: "Injectable PEGylated fibrinogen cell-laden microparticles made with a continuous solvent- and oil-free preparation method", ACTA BIOMATERIALIA, vol. 13, 18 novembre 2014 (2014-11-18), pages 78-87, XP055225933, NL ISSN: 1742-7061, DOI: 10.1016/j.actbio.2014.11.013

## Description

L'invention concerne une matrice en forme de bille, un procédé de préparation d'une telle matrice ainsi que son utilisation comme support pour cellules.

L'invention s'applique au domaine de la culture cellulaire et notamment pour l'expansion de cellules utilisées dans le cadre de la thérapie cellulaire, en bioproduction pour produire des molécules bioactives ou encore en recherche et développement comme modèles cellulaires. De telles cellules sont par exemple des cellules primaires, des cellules souches pluripotentes, des lignées de cellules souches ou des lignées cellulaires immortalisées.

En particulier, la thérapie cellulaire, basée sur l'utilisation de cellules souches adultes, consiste à remplacer et régénérer des cellules d'un tissu malade en administrant des cellules saines et fonctionnelles qui ont été sélectionnées, amplifiées et/ou modifiées ex vivo.

L'étape d'expansion cellulaire est nécessaire puisque les différentes sources de cellules souches ne permettent pas d'obtenir une quantité suffisante de cellules pour une utilisation clinique.

Actuellement, les cellules souches adhérentes sont cultivées dans des systèmes à deux dimensions, notamment dans des récipients de culture en plastique.

Pour obtenir une plus grande quantité de cellules, il est nécessaire d'utiliser une plus grande surface de culture, par exemple en utilisant un plus grand nombre de récipients de culture, en augmentant la taille des récipients de culture ou en utilisant des récipients de culture à plusieurs étages comme le CellStack™ (Corning, USA). Mais dans ces cas, la manipulation des récipients de culture devient plus laborieuse.

Des systèmes de culture en trois dimensions ont donc également été testés, utilisant notamment des microsupports en suspension.

Par exemple, le document US 2012/0009645 propose une méthode pour cultiver les cellules souches pluripotentes (cellules souches embryonnaires, cellules souche pluripotentes induites) sur des microbilles, en présence d'un inhibiteur ROCK. Les microbilles sont par exemple des billes de cellulose D53 ou des billes Cytodex 1 ou 3.

Cependant, l'utilisation de tels microsupports synthétiques présente plusieurs inconvénients. D'abord, ces microsupports possèdent un faible taux d'ensemencement des cellules (inférieur à 30%) entraînant dès le départ du processus d'expansion une perte massive de cellules d'intérêt. Ensuite, le détachement des cellules de leur support et la séparation support-cellules avant injection chez le patient reste un problème de taille. Enfin, ces microsupports synthétiques libèrent, lors de leur utilisation, des particules plastiques qui ne peuvent pas être bloquées et peuvent donc être injectées au patient en même temps que les cellules d'intérêts.

Pour pallier au problème d'adhérence des cellules, il a été proposé de revêtir les microsupports par des molécules. Cependant, ces molécules sont souvent chères et/ou d'origine animale ce qui présente un problème de sécurité biologique.

Par exemple, le document US 2011/0111498 propose une méthode pour cultiver des cellules souches (cellules souches mésenchymateuses, cellules souches embryonnaires, ou cellules souches pluripotentes induites) sur des microsupports en suspension. Les microsupports sont par exemple des billes synthétiques (Toyopearl, Cytodex, DE52) ayant une charge positive et un revêtement de type Matrigel™, laminine ou acide hyaluronique capable de supporter la croissance des cellules.

Le document WO 2009/134197 décrit également un microsupport, tel qu'une bille Cytodex, pour la culture de cellules, revêtu d'un hydrolysat de protéines de plante tel qu'un hydrolysat de peptones de soja. Ce microsupport possède l'avantage de ne pas comprendre de protéines animales.

Le document WO 2011/017050 propose un microsupport sans protéine d'origine animale pour la culture de cellules ayant un revêtement polymère (p.ex. méthacrylate gonflable) sur lequel est greffé un polypeptide comprenant la séquence RGD pour favoriser l'attachement des cellules.

Il existe également des microsupports de cellules à base de composants naturels.

Par exemple, le document EP 1 015 570, issu de la demande internationale de brevet WO 99/15637, propose des microbilles de fibrin(ogène) comme support pour cellules. Ces microbilles présentent un degré élevé de réticulation du fibrin(ogène) obtenu lors de la préparation des microbilles à partir de fibrinogène, thrombine et facteur XIII. Cependant, la thrombine utilisée comme agent de réticulation est exogène, ce qui augmente la difficulté de préparation et le risque biologique.

Dans le document Eibes G., et al. (Maximizing the ex vivo expansion of human mesenchymal stem cells using a microcarrier-based stirred culture system. Journal of biotechnology, 2010, vol. 146, no 4, p. 194-197), il est montré que des billes microporeuses de gélatine (Cultispher S) sont capables d'adhérer et de faire proliférer des cellules souches mésenchymateuses.

Enfin, le document Oliveira M.B., et al. (Injectable PEGylated fibrinogen cell-laden microparticles made with a continuous solvent- and oil-free preparation method. Acta Biomaterialia, vol. 13, 18 novembre 2014, p. 78-87), divulgue des microparticules de fibrinogène PEGylé. Ces microparticules, obtenues par la mise en oeuvre d'une méthode de préparation en continue par irradiation UV et en l'absence de solvant et d'huile, permet la culture cellulaire 3D et fournit une plateforme pour l'encapsulation des cellules à haute teneur en eau.

L'invention propose une matrice biocompatible tridimensionnelle pour cellules permettant d'obtenir un taux d'adhésion de plus de 75%. Cette matrice est en outre facilement dégradable pour pouvoir récupérer les cellules. La matrice possède de plus l'avantage de pouvoir être préparée à partir d'un produit biologique unique, sans nécessiter de protéines ou autres substances exogènes.

A cet effet et selon un premier aspect, l'invention concerne une matrice en forme de bille comprenant du fibrinogène réticulé, ladite matrice étant exempte de fibrine.

Selon un deuxième aspect, l'invention concerne un procédé de préparation d'une matrice selon le premier aspect, ledit procédé comprenant les étapes suivantes :
(a) la fourniture d'une composition initiale comprenant du fibrinogène et un facteur plaquettaire,
(b) l'introduction de ladite composition initiale dans une huile chauffée à une température comprise entre 50 et 80°C de sorte à former une émulsion,
(c) le mélange de l'émulsion ainsi obtenue à une température comprise entre 50 et 80°C jusqu'à l'obtention d'une matrice sous forme de bille, et
(d) l'isolement de la matrice ainsi obtenue.

Selon un troisième aspect, l'invention porte sur l'utilisation d'une matrice selon le premier aspect comme support pour cellules.

D'autres objets et avantages apparaîtront au cours de la description qui suit.

Les figures 1 et 2 représentent chacune une photographie en microscopie électronique à balayage d'une matrice poreuse selon l'invention.

La figure 3 représente une photographie en microscopie optique (x20) de matrices selon l'invention dans un milieu de culture et en présence de cellules.

Selon un premier aspect, l'invention concerne une matrice en forme de bille comprenant du fibrinogène réticulé. La matrice est notamment exempte de fibrine.

Par '"matrice", on désigne une structure tridimensionnelle solide ou semi-solide sur laquelle ou à l'intérieur de laquelle des cellules peuvent adhérer et/ou se multiplier.

La matrice de l'invention est sous forme de bille, c'est-à-dire qu'elle est sphérique ou sphéroïde. Elle peut avoir une forme régulière ou irrégulière.

La matrice est exempte de fibrine et comprend du fibrinogène réticulé ce qui permet d'obtenir des matrices mécaniquement stables et non dégradées pendant la culture de cellules.

Le fibrinogène est une protéine soluble présente dans le plasma. La protéine de fibrinogène est constituée de trois paires de chaînes peptidiques homologues liées entre elles par des ponts disulfures.

Le fibrinogène réticulé est du fibrinogène dans lequel au moins deux sites des chaînes polypeptidiques sont liés entre eux. La réticulation est intramoléculaire, les sites liés appartiennent alors à la même molécule de fibrinogène et/ou extra-moléculaire, deux ou plus molécules de fibrinogène sont alors liées entre elles. Le fibrinogène réticulé est insoluble dans l'eau et les solvants organiques.

*In vivo,* lors de la coagulation plasmatique, le fibrinogène est clivé par l'action de la thrombine en monomères de fibrine. Les monomères de fibrine vont ensuite se polymériser en fibrine, instable. Le facteur XIIIA va finalement permettre la réticulation du polymère de fibrine de sorte à le stabiliser.

La réticulation du fibrinogène est par conséquent un processus non naturel qui a été obtenu de façon surprenante par la demanderesse à partir d'une composition initiale particulière. La matrice de fibrinogène réticulé de l'invention est une matrice synthétique de fibrinogène qui possède notamment une structure tridimensionnelle non naturelle en forme de bille et est exempte de fibrine.

Avantageusement, le fibrinogène contenu dans la matrice n'a pas été fonctionnalisé physiquement et/ou chimiquement, par exemple par pégylation. Le fibrinogène contenu dans la composition initiale est un fibrinogène naturel qui n'a pas été modifié par l'homme.

Selon une réalisation particulière, la composition initiale comprend du fibrinogène soluble. Plus particulièrement, la composition initiale comprend du plasma qui est une source de fibrinogène. Encore plus particulièrement, le plasma est du plasma humain.

Notamment, le fibrinogène est réticulé par l'intermédiaire d'un facteur plaquettaire.

Par facteur plaquettaire, on désigne une ou plusieurs substances contenues ou secrétées par les plaquettes. Notamment, le facteur plaquettaire est une substance contenue dans un lysat plaquettaire.

Par lysat plaquettaire, on désigne le produit de la lyse des plaquettes, c'est-à-dire le produit obtenu après désintégration de la membrane cellulaire qui conduit à la libération des molécules (facteurs de croissance, cytokines) normalement contenues à l'intérieur des plaquettes.

La lyse des plaquettes est par exemple obtenue par un ou plusieurs cycles de congélation/décongélation, par utilisation d'ultra-sons ou par traitement par solvant/détergent.

La réticulation du fibrinogène est rendue possible par la présence d'un facteur plaquettaire. Le procédé de préparation décrit ci-après réalisé à partir d'une composition initiale de plasma sans plaquettes à la place d'une composition initiale de lysat de plaquettes n'a pas aboutit à la formation de matrices. La réticulation du fibrinogène sans thrombine exogène et donc sans formation de fibrine est due à la présence d'une ou plusieurs substances du lysat plaquettaire.

En particulier, la réticulation se produit sans avoir besoin d'ajouter ni une substance chimique telle que du glutaraldéhyde ou un dérivé de carbodiimide ni une substance xénogène tel que la thrombine non humaine, la génipine ou un sucre.

La matrice possède ainsi l'avantage de ne contenir que des protéines d'origine humaine. La matrice est ainsi biocompatible, c'est-à-dire non toxique vis-à-vis des cellules, tissus et organes.

Selon une réalisation, la matrice possède un diamètre compris entre 20 µm et 2 mm, notamment entre 50 µm et 1 000 µm. Par exemple la matrice possède un diamètre compris entre 200 et 800 µm.

En particulier, la matrice de l'invention est une matrice poreuse, c'est-à-dire qu'elle possède une pluralité d'interstices ou d'orifices dans sa structure.

Notamment, la matrice selon l'invention présente des pores ayant un diamètre inférieur à 1 µm.

La porosité de la matrice se vérifie grâce aux techniques de microscopie électronique.

La matrice de l'invention étant à base de fibrinogène, elle possède la propriété d'adhésion cellulaire.

Du fait de la faible taille des pores de la matrice, les cellules qui y adhérent restent à sa surface et ne migrent pas à l'intérieur. Cette matrice poreuse est également capable d'adsorber des agents bioactifs de petite taille telle que des facteurs de croissance, des cytokines, des hormones ou autres principes actifs.

Selon une réalisation particulière, la matrice comprend en outre au moins un agent bioactif tel qu'une cellule, une protéine, par exemple un facteur de croissance, un médicament, une hormone, une cytokine ou une combinaison de ceux-ci.

L'agent bioactif est encapsulé, absorbé, adsorbé et/ou lié de façon covalente à la matrice.

Dans un exemple particulier, la matrice comprend des cellules primaires et/ou des lignées cellulaires. Des exemples de telles cellules sont des cellules souches, des fibroblastes, des cellules endothéliales, des chondrocytes, des cellules de neuroblastome, des cellules rénales, des cellules hépatiques, des cellules pancréatiques, des cellules thyroïdiennes, des cellules gliales, des cellules musculaires, des cellules de carcinome mammaire de souris et des combinaisons de celles-ci. Encore plus particulièrement, les cellules sont des cellules CHO (Chinese hamster ovary, ovaires de hamster de Chine), des cellules Vero ou des cellules souches mésenchymateuses.

Dans un autre exemple particulier, la matrice ne comprend pas de cellules souches embryonnaires humaines.

Selon une réalisation spécifique, les cellules sont infectées par un virus, un gêne exprimant une protéine recombinante d'intérêt ou un ADN exogène.

Notamment, la matrice comprend en outre au moins un facteur de croissance tel que l'IGF-1 (insulin-like growth factor-1, facteur de croissance 1 ressemblant à l'insuline). Plus particulièrement, le facteur de croissance est endogène, c'est-à-dire qu'il est compris dans la composition initiale comprenant le fibrinogène destiné à former la matrice.

La concentration en facteur(s) de croissance endogène(s) dans la matrice varie en fonction du procédé de préparation et/ou de la concentration initiale en facteur(s) de croissance dans la composition initiale.

Les matrices de l'invention ont l'avantage d'être complètement dégradables par action enzymatique et/ou chimique. Cette propriété facilite la récupération des cellules après leur expansion.

Par exemple, des agents enzymatiques ou non enzymatiques de dissociation cellulaire tels que la trypsine, la combinaison trypsine-EDTA, l'Accutase™ (Chemicon) ou la TrypLE™ (Gibco), sont utilisés pour dégrader les matrices et en détacher ainsi les cellules.

Selon un autre aspect, l'invention concerne un procédé de préparation d'une matrice selon le premier aspect, comprenant les étapes suivantes :
(a) la fourniture d'une composition initiale comprenant du fibrinogène et un facteur plaquettaire,
(b) l'introduction de ladite composition initiale dans une huile chaude à une température comprise entre 50 et 80°C de sorte à former une émulsion,
(c) le mélange de l'émulsion ainsi obtenue à une température comprise entre 50 et 80°C jusqu'à l'obtention d'une matrice sous forme de bille, et
(d) l'isolement de la matrice ainsi obtenue.

Le produit de départ du procédé est une composition initiale comprenant du fibrinogène et un facteur plaquettaire. Notamment, la composition initiale comprend du plasma comme source de fibrinogène et un lysat plaquettaire comme source de facteur plaquettaire. Par ailleurs, la composition initiale ne comprend pas de thombine exogène qui simplifie la préparation et diminue le risque biologique.

Avantageusement, la source de plasma et la source de lysat plaquettaire sont communes. Dans ce cas, la composition initiale est un lysat plaquettaire obtenu par lyse de plaquettes en suspension dans du plasma. Par exemple, la composition initiale est un lysat de plaquettes obtenu par lyse d'un ou plusieurs concentrés de plaquettes ou d'un ou plusieurs plasmas riche en plaquettes.

Selon une variante, le concentré plaquettaire comprend une solution additive de conservation des plaquettes. Dans ce cas, la quantité de plasma et donc de fibrinogène dans le concentré plaquettaire doit rester suffisante pour permettre la formation des matrices.

Afin de favoriser l'organisation sphérique et de réduire l'agrégation des matrices entre elles, la composition initiale comprend en outre un agent tensioactif.

Par "agent tensioactif", on désigne une molécule amphiphile, c'est-à-dire qui présente deux parties de polarité différente, l'une lipophile (miscible dans l'huile) et apolaire, l'autre hydrophile (miscible dans l'eau) et polaire.

L'agent tensioactif a pour fonction de faciliter la formation de l'émulsion, de la stabiliser et de favoriser la dispersion des gouttelettes formées.

L'agent tensioactif est un agent anionique, cationique, amphotère ou non ionique. Des exemples d'agents tensioactifs sont notamment le sodium dodécyl sulfate, le Triton X-100, le Tween 20, le Tween 80 et les combinaisons de ceux-ci.

En particulier, la composition comprend entre 0,5 et 20% d'agent tensioactif, notamment entre 1 et 5%.

Le procédé comprend ensuite l'introduction de la composition initiale dans une huile chauffée à une température comprise entre 50 et 80°C, de sorte à former une émulsion. Des petites gouttelettes vont alors se former et être dispersées dans l'huile chaude.

Selon une réalisation particulière, l'huile est une huile animale ou végétale, notamment végétale telle qu'une huile de tournesol, d'olive ou de colza.

On suppose que sous l'influence de la chaleur, des sites de liaison non accessibles de la protéine de fibrinogène vont être découverts et permettre la réticulation, de sorte à rendre le fibrinogène insoluble dans l'eau et dans les solvants organiques.

Dans le cas où la composition initiale est un lysat plaquettaire obtenu par lyse des plaquettes en suspension dans le plasma, la réticulation du fibrinogène est produite de façon intrinsèque, c'est-à-dire sans avoir recours à une substance extérieure.

L'émulsion ainsi obtenue est ensuite mélangée pour former la matrice.

La façon de mélanger l'émulsion a un impact sur les matrices. Notamment, le mélange de l'émulsion est réalisé par agitation à une vitesse comprise entre 100 et 1500 cycles par minutes, notamment supérieure à 700 cycles par minutes. Plus la vitesse d'agitation sera élevée, plus petites seront les matrices.

Le mélange est réalisé pendant une période de temps comprise entre 2 heures et 10 heures, notamment entre 3 et 5 heures.

En variant la quantité et le type d'agent tensioactif et/ou en variant la vitesse d'agitation, il est possible de contrôler la taille des matrices.

L'étape d'isolement des matrices formées est réalisée par filtration puis lavages successifs des matrices à l'aide d'un solvant organique afin d'éliminer les traces d'huile.

Le procédé aboutit à la formation de matrices de différente taille. En variante, le procédé comprend, après l'isolement des matrices, l'étape de filtrer les matrices au travers de tamis de taille décroissante afin d'obtenir des matrices calibrées.

Le procédé pour obtenir les matrices de l'invention est un procédé non naturel qui nécessite notamment un apport de chaleur supérieur à 50°C.

Les matrices obtenues par ce procédé à partir d'un lysat plaquettaire sont poreuses et essentiellement constituées de fibrinogène réticulé et sont exemptes de fibrine.

Selon un troisième aspect, l'invention concerne l'utilisation d'une matrice selon le premier aspect comme support pour cellules.

Pour la culture de cellules, les matrices sont mises en suspension dans un milieu de culture contenu dans un récipient de culture. Les cellules à cultiver sont ensuite ensemencées, c'est-à-dire mises en contact avec les matrices.

Par exemple, les matrices sont utilisées pour l'expansion cellulaire, la production de protéines d'intérêt et la régénération cellulaire.

Les matrices étant biocompatibles, elles peuvent être utilisées in vivo avec ou sans agent bioactif.

Un avantage particulier des matrices de l'invention est son pouvoir d'adhésion cellulaire très élevé. En effet, plus de 75% des cellules adhérent aux matrices lors de l'étape d'ensemencement.

D'autres applications pour les matrices de l'invention sont, par exemple, la régénération de tissus, la cicatrisation, la libération contrôlée d'un médicament ou encore la sélection de cellules souches.

### Exemple

### Fabrication des billes

200 millilitres d'huile de tournesol est mise à chauffer dans un récipient de 500ml sous agitation magnétique jusqu'à atteindre 65°C. 50 ml de lysat plaquettaire est alors ajouté dans l'huile chaude. La solution ainsi obtenue est maintenue sous agitation à 65°C jusqu'à apparition des billes. Le chauffage de la préparation est ensuite arrêté et l'agitation est maintenue jusqu'à retour à température ambiante. La préparation est ensuite filtrée sur des tamis de taille calibrée et les billes récupérées sont ensuite lavées trois fois par des solvants organiques (xylène, hexane et éthanol) et reprises dans une solution saline.

De par leur structure particulière, les billes peuvent également être conservées sous forme de poudre. Les billes peuvent ensuite être conservées à température ambiante ou à 4°C pour une conservation plus longue.

Les billes ainsi obtenues sont sphériques et possèdent un diamètre allant de 30 µm à 2 000 µm. Par tamisage, il est possible d'obtenir une population de billes de taille voulue.

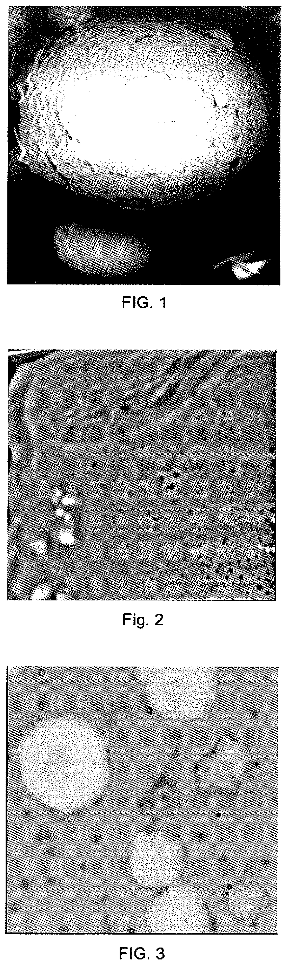

Les analyses en microscopie électronique à balayage ont permis de mettre en évidence une structure compacte permettant l'adhésion cellulaire (figures 1 et 2).

### Ensemencement des billes par les cellules

Les billes en solution ont été mises en contact avec un nombre connu de cellules souches mésenchymateuses dans un petit volume de milieu de culture complet (10ml). Les cellules adhèrent aux billes (figure 3).

Après 4 heures de contact à l'incubateur à 37°C, le milieu de culture (sans les billes) a été collecté et centrifugé. Le nombre de cellules non adhérées aux billes se retrouvant dans le surnageant a été compté permettant de déterminer un taux d'adhésion cellulaire qui se situe à 75%.

### Digestion des billes pour la récupération des cellules

Les billes chargées avec les cellules ont été maintenues en culture afin d'obtenir un nombre de cellules suffisant. A la fin de la période de culture cellulaire, les complexes billes/cellules sont récupérés par centrifugation de la totalité du milieu de culture. Le culot récupéré, constitué des complexes billes/cellules, est lavé 2 fois au PBS. A la suite des lavages, une enzyme (TrypLE™) permettant la digestion totale des billes sans entraîner de mortalité cellulaire, est ajoutée sur le culot et placé 20 minutes à 37°C. Après digestion totale des billes, la solution est centrifugée et les cellules récupérées peuvent ensuite être utilisées.

### Taux de prolifération cellulaire

Des tests de prolifération cellulaire sur des billes selon l'invention ont été effectués en comparaison avec des billes de polystyrène.

Une lignée cellulaire murine et des cellules primaires humaines ont été ensemencées à la même densité sur les 2 types de billes. La quantité de cellules non adhérées a été évaluée afin de connaître précisément la quantité de cellules adhérées.

Les cellules sur les billes ont été maintenues en culture durant 10 jours avec ajout de milieu frais tous les 3 jours. En fin de culture, les complexes billes/cellules ont été récupérés et la quantité totale de cellules générées a été évaluée.

Les tests nous ont permis de montrer, sur les billes selon l'invention, une amplification cellulaire de 23 fois pour la lignée cellulaire murine (contre 30 fois pour les billes de polystyrène) et de 14 fois pour la lignée primaire humaine (contre 12 fois pour les billes de polystyrène)

### Caractérisation des billes

Les billes, après leur fabrication, ont été digérées afin d'étudier précisément leur composition. Des dosages immunologiques ont permis de déterminer que les billes selon l'invention étaient constituées majoritairement de fibrinogène (en moyenne présent à 100 µg/ml). Les dosages ont permis de montrer la présence, au sein des billes selon l'invention, d'IGF-1 à une concentration de 0.3 pg/ml.

La présence de fibrine n'a pas été détectée au sein des billes.

## Revendications

1. Matrice en forme de bille comprenant du fibrinogène réticulé **caractérisée en ce qu'**elle exempte de fibrine.

2. Matrice selon la revendication 1, **caractérisée en ce qu'**elle est poreuse.

3. Matrice selon la revendication 2, **caractérisée en ce qu'**elle présente des pores ayant un diamètre inférieur à 1 µm.

4. Matrice selon l'une quelconque des revendications 1 à 3, **caractérisée en ce que** le fibrinogène est réticulé par l'intermédiaire d'un facteur plaquettaire.

5. Matrice selon l'une quelconque des revendications 1 à 4, **caractérisée en ce qu'**elle possède un diamètre compris entre 20 µm et 2 mm, notamment entre 50 µm et 1 000 µm.

6. Matrice selon l'une quelconque des revendications 1 à 5, **caractérisée en ce qu'**elle comprend en outre au moins un agent bioactif tel qu'une cellule, une protéine, un médicament, une hormone, une cytokine ou une combinaison de ceux-ci.

7. Matrice selon la revendication 6, **caractérisée en ce que** la protéine est un facteur de croissance tel que l'IGF-1.

8. Matrice selon l'une quelconque des revendications 1 à 7 sur la surface de laquelle des cellules sont adhérées.

9. Procédé de préparation d'une matrice selon l'une quelconque des revendications 1 à 8, comprenant les étapes suivantes :
(a) la fourniture d'une composition initiale comprenant du fibrinogène et un facteur plaquettaire,
(b) l'introduction de ladite composition initiale dans une huile chauffée à une température comprise entre 50 et 80°C de sorte à former une émulsion,
(c) le mélange de l'émulsion ainsi obtenue à une température comprise entre 50 et 80°C jusqu'à l'obtention d'une matrice sous forme de bille, et
(d) l'isolement de la matrice ainsi obtenue.

10. Procédé selon la revendication 9 **caractérisé en ce que** la composition initiale est un lysat plaquettaire obtenu par lyse de plaquettes en suspension dans du plasma.

11. Procédé selon la revendication 10, **caractérisé en ce que** le lysat plaquettaire est obtenu à partir d'un ou plusieurs concentrés de plaquettes.

12. Procédé selon l'une quelconque des revendications 9 à 11, **caractérisé en ce que** la composition initiale comprend en outre un agent tensioactif.

13. Procédé selon la revendication 12, **caractérisé en ce que** la composition initiale comprend entre 0,5 et 20% d'agent tensioactif, notamment entre 1 et 5%.

14. Procédé selon l'une quelconque des revendications 9 à 13, **caractérisé en ce que** le mélange de l'émulsion est réalisé par agitation à une vitesse comprise entre 100 et 1 500 cycles par minutes.

15. Procédé selon l'une quelconque des revendications 9 à 14, **caractérisé en ce que** l'huile est une huile végétale, notamment de l'huile d'olive.

16. Procédé selon l'une quelconque des revendications 9 à 15, **caractérisé en ce que** le mélange de l'émulsion est réalisé pendant une période de temps comprise entre 2 heures et 10 heures.

17. Utilisation d'un matrice selon l'une quelconque des revendications 1 à 8 comme support pour cellules.

## Patentansprüche

1. Matrix in Kugelform, umfassend vernetztes Fibrinogen, **dadurch gekennzeichnet, dass** sie frei von Fibrin ist.

2. Matrix nach Anspruch 1, **dadurch gekennzeichnet, dass** sie porös ist.

3. Matrix nach Anspruch 2, **dadurch gekennzeichnet, dass** sie Poren mit einem Durchmesser kleiner als 1 µm aufweist.

4. Matrix nach einem der Ansprüche 1 bis 3, **dadurch gekennzeichnet, dass** das Fibrinogen über einen Thrombozytenfaktor vernetzt ist.

5. Matrix nach einem der Ansprüche 1 bis 4, **dadurch gekennzeichnet, dass** sie einen Durchmesser zwischen 20 µm und 2 mm, insbesondere zwischen 50 µm und 1000 µm aufweist.

6. Matrix nach einem der Ansprüche 1 bis 5, **dadurch gekennzeichnet, dass** sie ferner mindestens ein bioaktives Mittel wie etwa eine Zelle, ein Protein, ein Arzneimittel, ein Hormon, ein Zytokin oder eine Kombination davon umfasst.

7. Matrix nach Anspruch 6, **dadurch gekennzeichnet, dass** das Protein ein Wachstumsfaktor wie IGF-1 ist.

8. Matrix nach einem der Ansprüche 1 bis 7, an deren Oberfläche Zellen anhaften.

9. Verfahren zum Herstellen einer Matrix nach einem der Ansprüche 1 bis 8, umfassend die folgenden Schritte:
(a) Bereitstellen einer Ausgangszusammensetzung, umfassend Fibrinogen und einen Thrombozytenfaktor,
(b) Injizieren der Ausgangszusammensetzung in ein auf eine Temperatur von 50°C bis 80°C erhitztes Öl, um eine Emulsion zu bilden,
(c) Mischen der so erhaltenen Emulsion bei einer Temperatur von 50 °C bis 80 °C, bis eine Matrix in Kugelform erhalten wird, und
(d) Isolieren der so erhaltenen Matrix.

10. Verfahren nach Anspruch 9, **dadurch gekennzeichnet, dass** die Ausgangszusammensetzung ein Thrombozytenlysat ist, das durch Lyse von Thrombozyten in Suspension in Plasma erhalten wird.

11. Verfahren nach Anspruch 10, **dadurch gekennzeichnet, dass** das Thrombozytenlysat aus einem oder mehreren Thrombozytenkonzentraten erhalten wird.

12. Verfahren nach einem der Ansprüche 9 bis 11, **dadurch gekennzeichnet, dass** die Ausgangszusammensetzung ferner ein Tensid enthält.

13. Verfahren nach Anspruch 12, **dadurch gekennzeichnet, dass** die Zusammensetzung zwischen 0,5 und 20 % Tensid, insbesondere zwischen 1 und 5 % umfasst.

14. Verfahren nach einem der Ansprüche 9 bis 13, **dadurch gekennzeichnet, dass** das Mischen der Emulsion durch Rühren bei einer Geschwindigkeit zwischen 100 und 1500 Zyklen pro Minute erfolgt.

15. Verfahren nach einem der Ansprüche 9 bis 14, **dadurch gekennzeichnet, dass** das Öl ein Pflanzenöl, insbesondere Olivenöl ist.

16. Verfahren nach einem der Ansprüche 9 bis 15, **dadurch gekennzeichnet, dass** das Mischen der Emulsion über einen Zeitraum zwischen 2 Stunden und 10 Stunden erfolgt.

17. Verwendung einer Matrix nach einem der Ansprüche 1 bis 8 als Zellträger.

## Claims

1. A matrix in ball form comprising cross-linked fibrinogen, **characterized in that** it is free from fibrin.

2. The matrix according to claim 1, **characterized in that** it is porous.

3. The matrix according to claim 2, **characterized in that** it has pores with a diameter smaller than 1 µm.

4. The matrix according to any one of claims 1 to 3, **characterized in that** the fibrinogen is cross-linked via a platelet factor.

5. The matrix according to any one of claims 1 to 4, **characterized in that** it has a diameter comprised between 20 µm and 2 mm, in particular between 50 µm and 1000 µm.

6. The matrix according to any one of claims 1 to 5, **characterized in that** it further comprises at least one bioactive agent such as a cell, a protein, a medicinal product, a hormone, a cytokine or a combination thereof.

7. The matrix according to claim 6, **characterized in that** the protein is a growth factor such as IGF-1.

8. The matrix according to any one of claims 1 to 7, on the surface of which cells are adhered.

9. A method for preparing a matrix according to any one of claims 1 to 8, comprising the following steps:
(a) providing an initial composition comprising fibrinogen and a platelet factor,
(b) injecting said initial composition into an oil heated to a temperature of 50°C to 80°C so as to form an emulsion,
(c) mixing the emulsion thus obtained at a temperature of 50°C to 80°C until a matrix in ball form is obtained, and
(d) isolating the matrix thus obtained.

10. The method according to claim 9, **characterized in that** the initial composition is a platelet lysate obtained by lysis of platelets in suspension in plasma.

11. The method according to claim 10, **characterized in that** the platelet lysate is obtained from one or several platelet concentrates.

12. The method according to any one of claims 9 to 11, **characterized in that** the initial composition further comprises a surfactant agent.

13. The method according to claim 12, **characterized in that** the composition comprises between 0.5 and 20% surfactant agent, in particular between 1 and 5%.

14. The method according to any one of claims 9 to 13, **characterized in that** the mixing of the emulsion is done by agitation at a speed comprised between 100 and 1500 cycles per minute.

15. The method according to any one of claims 9 to 14, **characterized in that** the oil is a plant oil, in particular olive oil.

16. The method according to any one of claims 9 to 15, **characterized in that** the mixing of the emulsion is done for a length of time comprised between 2 hours and 10 hours.

17. A use of a matrix according to any one of claims 1 to 8 as a cell carrier.
